# EUROPÄISCHE PATENTANMELDUNG

(11) **EP 1 175 870 A1**
(43) Veröffentlichungstag der Anmeldung: **30.01.2002**
(21) Anmeldenummer: 00810667.6
(22) Anmeldetag: 26.07.2000
(51) Int. Cl.: A61B 17/16

(54) **Hohlbohrer zum Gewinnen von Knochenmaterial**

(71) Anmelder: Sulzer Orthopedics Ltd., 6340 Baar (CH)
(72) Erfinder: Frei, Heribert, 8404 Winterthur (CH); Jakob, Roland P., 1787 Môtier (CH); Reinmann, Andreas, 4542 Luterbach (CH)
(74) Vertreter: Sulzer Management AG

(57) **Zusammenfassung**

Mit der Erfindung werden Hohlbohrer zum Gewinnen von Knochenmaterial gezeigt, die einen rohrförmigen Schaft (1) und daran stirnseitig vorstehende Zähne (10) mit Schneiden (2) aufweisen, welche annähernd in radialer Richtung verlaufen. Dadurch, dass die Schneiden (2) einen positiven Spanwinkel (6) und einen kleinen Freiwinkel (4) grösser 3° aufweisen, sind nur geringe Vorschubkräfte notwendig und entsteht ein Schnittvorgang mit geringer Wärmeentwicklung, wobei eine Aufweitung (8) auf der Innenseite des Hohlbohrers zusätzlich den Kontakt und das Entstehen von Reibungswärme am freigeschnittenen Knochenstäbchen verhindert.

## Beschreibung

Die Erfindung handelt von einem Hohlbohrer zum Gewinnen von Knochenmaterial, der einen rohrförmigen Schaft und daran stirnseitig vorstehende Zähne mit Schneiden aufweist, welche annähernd in radialer Richtung verlaufen.

So zeigt die EP-A-0 824 893 eine scharfkantige Hülse mit einer umlaufenden ringförmigen Schneide, welche wie ein Locheisen mit Hammerschlägen in das Knorpel- und Knochengewebe eingeschlagen wird und anschliessend durch Vorwärts-Rückwärtsdrehen in einem Winkelbereich von 90° zu einem Abscheren eines freigeschnittenen Stäbchens führt.

Andere Autoren schlagen in Handbohrmaschinen einsetzbare Hohlbohrer vor. Die US 5,632,747 zeigt eine Bohrvorrichtung mit einer vorlaufenden Zentrierhilfe, um mit einem mit Zähnen versehenen Rohrstück ein Knochenstäbchen frei zu schneiden.

Beide Methoden haben Nachteile. Im ersten Fall verdrängt die Schneide, welche sich bis auf die Wandstärke der Hülse erweitert, Knochengewebe und schädigt Nachbarzonen. Auch mit einer sehr scharfen Schneide sind grössere Hammerschläge notwendig, die sich auf den Knochen übertragen. Im zweiten Fall haben eigene Versuche gezeigt, dass mit nicht fachgerechter Anwendung Temperaturanstiege bis über 30°C lokal im Knochengewebe erzeugt werden, die zu einer Schädigung des Knochengewebes führen können.

Die Erfindung hat die Aufgabe, einen einfachen und sicheren Hohlbohrer für Knochenmaterial zu schaffen. Diese Aufgabe wird mit den Merkmalen des unabhängigen Anspruchs1 erreicht, indem die Schneiden bezüglich einer zum Bohren vorgesehenen Drehrichtung einen Freiwinkel grösser als 3° und zu einer Senkrechten zur Drehrichtung einen positiven Spanwinkel aufweisen und indem der rohrförmige Schaft auf seiner Innenseite von einem Abstand zu den Schneiden bis zu einer maximalen Eindringtiefe eine Aufweitung mit einem Kreisdurchmesser D₂ aufweist, welcher grösser ist als der Innendurchmesser im Bereich der Zähne.

Die Geometrie der Schneiden bewirkt mit einem positiven Spanwinkel und mit einem begrenzten Freiwinkel, dass sich bei einer mittleren Vorschubkraft ein langsam drehender Hohlbohrer kontrolliert und langsam in das Knochenmaterial hineinzieht und einen stabförmigen Kern stehen lässt. An Haupt- und Nebenschneiden entsteht ein Schnittvorgang, der kaum zu Erwärmungen im Knochengewebe führt. Im Gegensatz zu hochtourigen Hohlbohrern, bei denen negative Spanwinkel gewählt werden müssen, um eine bestimmte Bohrtiefe sicher anfahren zu können, wird mit einem sehr langsam drehenden Hohlbohrer und positiven Spanwinkeln nur ein Minimum an Reibungswärme erzeugt. Vergleichsversuche haben ergeben, dass mit dem vorgeschlagenen Hohlbohrer Temperaturanstiege von 3° bis 5°C eingehalten werden können. Dadurch, dass sich der Innenraum hinter den Zähnen aufweitet, entsteht ein Freiraum zum frisch geschnittenen Knochenstäbchen, der eine Berührung und Erzeugung von Reibungswärme verhindert. Im weiteren wird durch die klaren Schnittbedingungen das an die Schnittzone angrenzende Knochenmaterial in Stäbchen und Knochen kaum beeinflusst. Ein Knochenstäbchen behält daher seine Festigkeit und wird nicht vorzeitig an seiner Basis abgeschert. Es können daher auch Stäbchen mit einem kleinen Durchmesser von wenigen Millimetern entnommen werden. Weitere Vorteile ergeben sich durch die Weiterbildung der Erfindung in den abhängigen Ansprüchen 2 bis 13. Wenn die Nebenschneiden ebenfalls scharfkantig ausgeführt sind, wird die Schnittarbeit recht klein. Der Freiwinkel und der Abstand der Schneiden bestimmen weitgehend den Vorschub pro Zahn, wenn ein positiver Spanwinkel gegeben ist. Mit vielen Zähnen am Umfang ist der Anteil der Nebenschneiden am Schnittvorgang gering und profitiert vom schwächenden Randeffekt der mit günstigeren Schnittwinkeln ausgestatteten Hauptschneide. Wenn die Höhe, mit der die Zähne vorstehen, entsprechend klein gewählt wird, beispielsweise zwischen 0,3 und 2 mm, lassen sich zwischen 10 und 20 Zähne mit kleinem Vorschub anbringen. Wenn zusätzlich der Abstand der Schneiden zur Aufweitung auf der Innenseite klein gehalten wird, beispielsweise kleiner als 4 mm, kann die Wärmeentwicklung auf der Innenseite sehr niedrig gehalten werden.

Eine kreiszylindrische, äussere Mantelfläche ergibt während des Bohrvorgangs eine genaue Führung. Dadurch, dass bei grösseren positiven Spanwinkeln beispielsweise bei 15° nur noch geringe Vorschubkräfte in Richtung der Drehachse notwendig sind, treten auch geringere Seitenkräfte durch Verkanten auf und somit auch geringere Reibungskräfte an der äusseren Mantelfläche.

Eine weitere Verbesserung können Spanabfuhrkanäle bilden, die von den Spanflächen bis zur Aufweitung der Innenseite reichen und das mehrfache Quetschen von bereits geschnittenem Material verringern.

Da die Schnittkräfte relativ gering sind, kann der Hohlbohrer direkt oder über einen Adapter mit einem Handantrieb versehen werden. Es genügt bereits ein T-förmiger Handgriff, der zusätzlich den Vorteil hat, dass durch das Umsetzen der Hand oder durch ein Rückwärtsdrehen eine Pause entsteht, die ein Abfliessen der beim Schneiden entstandenen Wärme begünstigt. Eine Handkurbel ist ebenfalls als Handantrieb möglich. Als Verbindung zwischen Handantrieb oder Adapter und Hohlbohrer ist eine Konusverbindung geeignet. Wenn der rohrförmige Schaft als Konushülse fortgesetzt ist, ergibt sich eine besondere einfache Herstellung des Hohlbohrers. Er kann einteilig ausgeführt werden und ist einfach zu reinigen und zu sterilisieren. Der Hohlbohrer kann aus Metall bestehen. Bei grossen Stückzahlen wäre es denkbar, den Hohtbohrer als einmaliges Wegwerfteil zu vermarkten. Er müsste dann an seinen Zähnen nicht unbedingt gehärtet sein und müsste auch nicht unbedingt aus Metall bestehen. Auch Hohlbohrer aus Kunststoff sind denkbar, wenn sie nur einmel verwendet werden.

Der hier beschriebene Hohlbohrer kann mit einem Bohrantrieb verbunden werden, der auf weniger als 3 Umdrehungen pro Sekunde begrenzt ist oder in einem Winkelbereich von weniger als 360° langsam, d.h. in mehr als einer halben Sekunde eine Vorwärts-Rückwärtsbewegung ausführt. Es ist auch möglich, dass der passende Bohrantrieb eine Stotterbewegung in einer Drehrichtung durchführt, die von regelmässigen Pausen zur Abkühlung gekennzeichnet ist. Bei allen bezüglich Drehzahl und Drehbewegung für den Hohlbohrer vorgesehenen Bohrantrieben ist es sinnvoll, eine mechanische Kodierung vorzusehen, die das Aufsetzen eines anderen zum Beispiel eines hochtourigen standartisierten Bohrantriebs verhindert.

Im folgenden wird die Erfindung anhand von Ausführungsbeispielen beschrieben. Es zeigen:
- Fig. 1: schematisch einen stark vergrösserten Längsschnitt durch einen erfindungsgemässen rechtsdrehenden Hohlbohrer,
- Fig. 2: schematisch eine sehr stark vergrösserte Ansicht auf die Zähne eines linksdrehenden Hohlbohrers,
- Fig. 3: schematisch einen Hohlbohrer mit Handantrieb und einem Adapter als Zwischenstück,
- Fig. 4: schematisch einen Hohlbohrer mit einem Adapter und mit einem Bohrantrieb,
- Fig. 5: schematisch einen Längsschnitt durch einen Hohlbohrer, der im Knochengewebe ein Stäbchen freigeschnitten hat, und
- Fig. 6: schematisch ein aus Kreisen und Geraden zusammengesetztes Zahnprofil.

Die Figuren zeigen Hohlbohrer zum Gewinnen von Knochenmaterial, die einen rohrförmigen Schaft 1 und daran stirnseitig vorstehende Zähne 10 mit Schneiden 2 aufweisen, welche annähernd in radialer Richtung verlaufen. Dadurch, dass die Schneiden 2 einen positiven Spanwinkel 6 und einen kleinen Freiwinkel 4 grösser als 3° aufweisen, sind nur geringe Vorschubkräfte notwendig und entsteht ein Schnittvorgang mit geringer Wärmeentwicklung, wobei eine Aufweitung 8 auf der Innenseite des Hohlbohrers zusätzlich den Kontakt und das Entstehen von Reibungswärme am freigeschnittenen Knochenstäbchen verhindert. Das Knochenstäbchen hat eine sehr geringe Kapazität Wärme zu speichern ohne dass die Temperatur austeigt.

In den nachfolgenden Ausführungsbeispielen werden gleiche Kennzeichen für gleiche Funktionen verwendet.

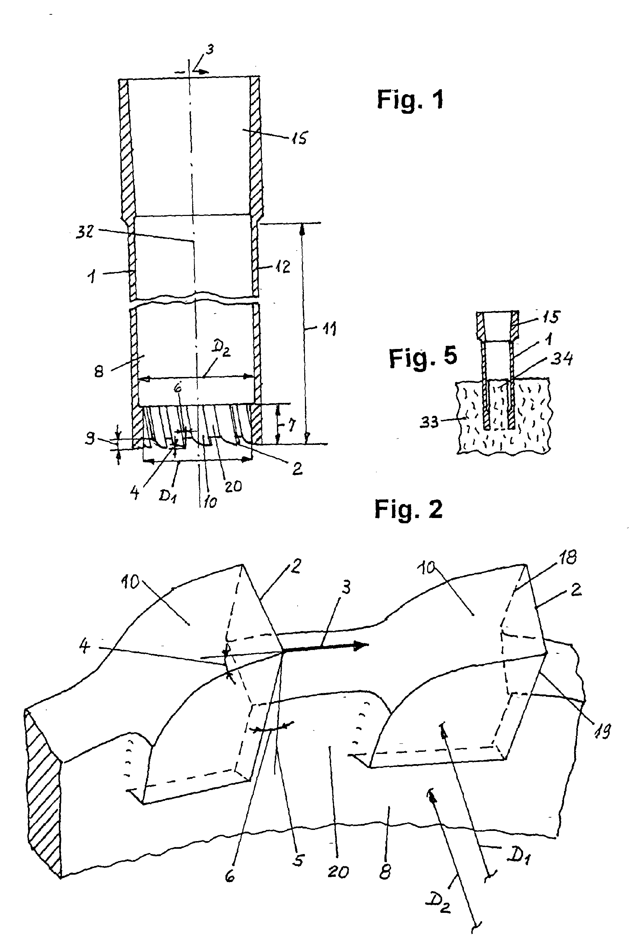

Der- Hohlbohrer in Figur 1 ist als Hülse ausgeführt, deren Oberteil eine Konushülse für eine Konusverbindung 15 bildet. Das Unterteil besteht aus einem rohrförmigen Schaft 1, dessen äussere Mantelfläche 12 auf einer maximalen Eindringtiefe11 kreisförmig zylindrisch ist. Auf der unteren Stirnseite stehen Zähne 10 mit Schneiden 2 um eine Höhe 9 vor. Der innere Durchmesser D₁ zwischen gegenüberliegenden Zähnen 10 wird bis zu einem Abstand 7 fortgesetzt, von dem aus eine Aufweitung 8 mit einem grösseren Durchmesser D₂ sich bis zur maximalen Eindringtiefe 11 anschliesst. Die Drehrichtung 3 des Hohlbohrers ist rechtsdrehend. Die Schneiden 2 bilden zur Drehrichtung 3 einen Freiwinkel 4, der grösser als 3° ist und zu einer Senkrechten 5 zur Drehrichtung 3 einen positiven Spanwinkel 6. Die Schneiden 2 sind also spitzwinklig und mit ihrer Spanfläche geneigt, um das geschnittene Knochenmaterial leichter abfliessen zu lassen.

In einer Fortsetzung der Spanflächen sind Spanabfuhrkanäle 20 angebracht, die einen Durchgang zur Aufweitung 8 bilden. Ein Beispiel für eine metallische Ausführung, die sehr dünnwandig ausgeführt werden kann, ergibt sich mit:

| | |
|---|---|
| Aussendurchmesser der Mantelfläche 12 | 7,3 mm |
| Innendurchmesser D₁ | 6,4 mm |
| Durchmesser D₂ der Aufweitung 8 | 6,6 mm |
| Höhe 9 der vorstehenden Zähne 10 | 0,6 mm |
| Abstand 7 der Aufweitung 8 | 2,5 mm |
| Anzahl der Zähne 10 | 14 |
| Zahnprofil analog zu Fig. 6 | |
| Konusverbindung 15 mit Gesamtkonuswinkel 2,85° | |

Es sind mehrere Grössen von Hohlbohrem vorgesehen, die Knochenstäbchen mit Durchmessern von 4 bis 10 mm erzeugen.

In Figur 2 sind die Verhältnisse an einem linksdrehenden Hohlbohrer im Bereich der stirnseitigen Zähne 10 aufgezeigt. Die Schneiden 2 besitzen einen spitzen Keilwinkel und verlaufen annähernd radial. Zur Drehrichtung 3 wird ein Freiwinkel 4 von mehr als 3° gebildet. Zu einer Senkrechten 5 zur Drehrichtung 3 besteht ein positiver Spanwinkel 6. Gegenüberliegende Zähne 10 bilden einen Durchmesser D₁, der in einer anschliessenden Aufweitung 8 auf einen Durchmesser D₂ übergeht. Eine Fortsetzung der Spanfläche bildet eine Seitenwand für einen Spanabfuhrkanal 20, der in die Aufweitung 8 führt. Die Seitenschneiden 18, 19 sind so scharfkantig wie möglich ausgeführt. Theoretisch wäre es möglich, die Spanfläche als Mulde auszuführen, um auch den Seitenschneiden 18, 19 einen positiven Spanwinkel zu ermöglichen.

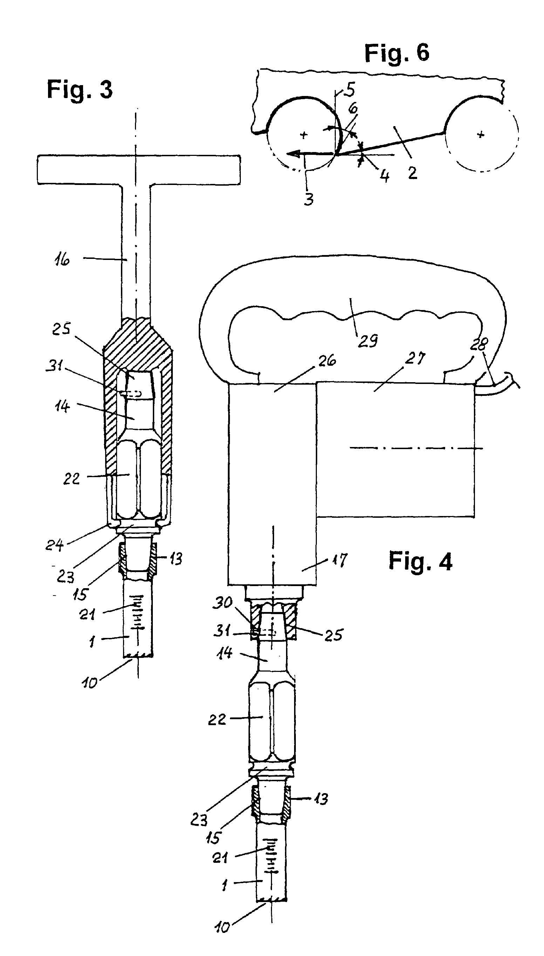

In Figur 3 ist der Hohlbohrer mit einer lösbaren Verbindung 13 in Form einer Konusverbindung 15 an einem Adapter 14 befestigt, der sowohl für einen Handantrieb 16, der hier aus einem T-förmigen Handgriff gebildet ist, als auch - wie später aus Figur4 ersichtlich ist - für einen maschinellen Bohrantrieb 17 geeignet ist. Der Mittelteil des Adapters 14 besteht aus einem Sechskant 22, in den der Handantrieb 16 eingreift, um ein notwendiges Drehmoment zu übertragen. Zur Uebertragung der Vorschubkraft stützt sich der Handantrieb 16 auf der Oberseite des Adapters 14 ab. Als Fortsetzung von einem passenden Aussensechskant bestitzt der Handantrieb 16 Federelemente 24, die in einer Nut 23 des Adapters 14 einrasten und die die lösbare Verbindung zum Adapter 14 sichern. Die Federelemente 24 lösen sich erst, wenn eine vorgegebene Auszugskraft überschritten wird. Der Adapter 14 besitzt an seiner Oberseite einen Morse-Konus 25 und einen Kodierstift 31, die für eine Anwendung eines Bohrantriebes gemäss Figur 4 notwendig sind und die so bemessen sind, dass sie das Aufsetzen eines Handantriebes 16 nicht hindern. Auf dem Hohlbohrer ist eine Tiefenskala 21 angebracht, um die Eindringtiefe zu kontrollieren.

Wenn auf einen Adapter 14, der auch für maschinelle Bohrantriebe geeignet ist, verzichtet wird, kann der Handantrieb 16 analog zum Adapter 14 direkt am Schaft 1 des Hohlbohrers befestigt werden. Dies ist durchaus zumutbar, da die Zerspanungskräfte mit der vorgeschlagenen Geometrie bei Handbetrieb klein sind.

Die kleinen Zerspanungskräfte und die geringe Zerspanungsarbeit bei kleinen Drehgeschwindigkeiten, die dem Handbetrieb entsprechen und eine Ueberhitzung des Knochenmaterials vermeiden, erlauben es, einen maschinellen Bohrantrieb 17 zu verwenden (Figur 4), der nur einen kleinen Leistungsverbrauch hat. Der Bohrantrieb 17 in Figur 4 besteht aus einem Elektromotor 27, der über ein Untersetzungsgetriebe 26 stark untersetzt ist. Der Bohrantrieb nimmt unter Einbezug einer mechanischen Kodierung, die hier aus einem Schlitz besteht, in den der Stift 31 einfährt, den Morse-Konus 25 auf. Ein Handgriff 29 dient zur Aufbringung der Vorschubkraft und als Abstützung zur Erzeugung des Drehmomentes beim bohren. Für den Elektromotor ist ein Kabelanschluss 28 eingezeichnet. Wegen der kleinen Zerspanungsleistung können stattdessen auswechselbare Akkumulatoren am Gerät angebracht sein.

Es gibt mehrere Möglichkeiten, den Motor anzusteuern, um zu einer gewünschten Drehgeschwindigkeit der Zähne 10 zu kommen.

Bei kontinuierlicher Drehung in einer Richtung dürfen nicht mehr als 3 Umdrehungen pro Sekunde möglich sein, um mit der vorgegebenen Geometrie grössere Temperaturerhöhungen zu verhindern. Noch besser ist der intermittierende Betrieb des Bohrantriebs, bei dem regelmässige Unterbrüche stattfinden. Die Pausen erlauben es auch viel besser, eine Tiefenskala 21 abzulesen.

Eine andere Betriebsart ist ein Vor- und Zurückdrehen in einem Winkelbereich kleiner 360°, wobei pro Zyklus mindestens eine halbe Sekunde aufgewendet werden sollte, um die Erwärmung klein zu halten.

In Figur 5 ist ein Hohlbohrer am Ende seiner Bohrtätigkeit gezeigt. Ein Knochenzapfen 34 ist aus dem gezeigten Knochenmaterial 33 freigeschnitten. Der Schaft 1 des Hohlbohrers ist so lang bemessen, dass unterschiedliche Bohrtiefen möglich sind. Falls die Konusverbindung 15 gelöst ist, lässt sich der Knochenzapfen 34 durch übertriebene Schwenkbewegungen mit dem rohrförmigen Schaft 1 an der Basis abbrechen und herausnehmen, wobei der abgelöste Knochenzapfen 34 nach oben oder nach unten aus dem Schaft 1 ausgestossen werden kann, was für die Entnahme von Gelenkknorpel interessant ist, um den Knorpel zu schonen.

In Figur 6 ist ein "aggressives" Zahnprofil gezeigt, welches aus herstelltechnischen Gründen aus Kreis- und Geradenabschnitten zusammengesetzt ist. Der Spanwinkel 6 ist positiv und der Freiwinkel 4 beträgt mehr als 3°.

## Patentansprüche

1. Hohlbohrer zum Gewinnen von Knochenmaterial, der einen rohrförmigen Schaft (1) und daran stirnseitig vorstehende Zähne (10) mit Schneiden (2) aufweist, welche annähernd in radialer Richtung verlaufen,
**dadurch gekennzeichnet, dass** die Schneiden (2) bezüglich einer zum Bohren vorgesehenen Drehrichtung (3) einen Freiwinkel (4) grösser 3° und zu einer Senkrechten (5) zur Drehrichtung (3) einen positiven Spanwinkel (6) aufweisen und dass der rohrförmige Schaft (1) auf seiner Innenseite von einem Abstand (7) zu den Schneiden (2) bis zu einer maximalen Eindringtiefe (11) eine Aufweitung (8) mit einem Kreisdurchmesser D₂ aufweist, welcher grösser ist als der Innendurchmesser D₁ im Bereich der Zähne (10).

2. Hohlbohrer nach Anspruch 1, **dadurch gekennzeichnet, dass** die Zähne (10) auf einer Höhe (9) von 0,3 bis 2 mm vorstehen.

3. Hohlbohrer nach einem der Ansprüche 1 oder 2, **dadurch gekennzeichnet, dass** der Abstand (7) der Schneiden (2) zu der Aufweitung (8) weniger als 4 mm beträgt.

4. Hohlbohrer nach einem der Ansprüche 1 bis 3, **dadurch gekennzeichnet, dass** er für die maximale Eindringtiefe (11) eine kreiszylindrische, äussere Mantelfläche aufweist.

5. Hohlbohrer nach einem der Ansprüche 1 bis 3, **dadurch gekennzeichnet, dass** Spanabfuhrkanäle (20) von der Spanfläche zur Aufweitung (8) vorgesehen sind.

6. Hohlbohrer nach einem der Ansprüche 1 bis 5, **dadurch gekennzeichnet, dass** der Schaft (1) über eine lösbare Verbindung (13) mit einem Adapter (14) verbindbar ist.

7. Hohlbohrer nach Anspruch 6, **dadurch gekennzeichnet, dass** die lösbare Verbindung (13) aus einer Konusverbindung besteht.

8. Hohlbohrer nach einem der Ansprüche 1 bis 7, **dadurch gekennzeichnet, dass** er aus Metall besteht.

9. Hohlbohrer nach einem der Ansprüche 1 bis 8, **dadurch gekennzeichnet, dass** er direkt oder über einen Adapter (14) mit einem Handantrieb (16) verbindbar ist, um manuell bohren zu können.

10. Hohlbohrer nach einem der Ansprüche 1 bis 8, **dadurch gekennzeichnet, dass** er direkt oder über einen Adapter (14) mit einem Bohrantrieb (17) verbindbar ist, der auf weniger als 3 Umdrehungen pro Sekunde begrenzt ist.

11. Hohlbohrer nach einem der Ansprüche 1 bis 8, **dadurch gekennzeichnet, dass** er direkt oder über einen Adapter (14) mit einem abwechselnd in beiden Drehrichtungen drehenden Bohrantrieb (17) verbindbar ist, dessen Drehbewegungen auf weniger als 360° beschränkt sind und dessen Vorwärts-Rückwärtsbewegung mindestens eine halbe Sekunde benötigt.

12. Hohlbohrer nach einem der Ansprüche 10 oder 11, **dadurch gekennzeichnet, dass** er direkt oder über den Adapter (14) mit dem Bohrantrieb (17) verbindbar ist, wobei die Verbindung eine mechanische Kodierung (30) enthält, die das Aufsetzen eines anderen, zum Beispiel eines standartisierten hochtourigen Bohrantriebs verhindert.

13. Hohlbohrer nach einem der Ansprüche 1 bis 11, **dadurch gekennzeichnet, dass** an die Schneiden (2) aussen und innen scharfkantige Nebenschneiden (18, 19) angrenzen.
